(19) Europäisches Patentamt
European Patent Office
Office européen des brevets

(11) **EP 2 710 348 B1**

(12) **EUROPÄISCHE PATENTSCHRIFT**

(45) Veröffentlichungstag und Bekanntmachung des Hinweises auf die Patenterteilung:
**31.08.2016   Patentblatt 2016/35**

(51) Int Cl.:
***G01N 21/27*** *(2006.01)*

(21) Anmeldenummer: **12762538.2**

(22) Anmeldetag: **16.07.2012**

(86) Internationale Anmeldenummer:
**PCT/EP2012/063864**

(87) Internationale Veröffentlichungsnummer:
**WO 2013/010970 (24.01.2013 Gazette 2013/04)**

(54) **VERFAHREN UND SYSTEM ZUM BESTIMMEN DER KONZENTRATION VON SUBSTANZEN IN KÖRPERFLÜSSIGKEITEN**

METHOD AND SYSTEM FOR DETERMINING THE CONCENTRATION OF SUBSTANCES IN BODILY FLUIDS

PROCÉDÉ ET SYSTÈME DE DÉTERMINATION DE LA CONCENTRATION DE SUBSTANCES DANS DES LIQUIDES CORPORELS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2011   EP 11174320**

(43) Veröffentlichungstag der Anmeldung:
**26.03.2014   Patentblatt 2014/13**

(73) Patentinhaber: **Siemens Healthcare Diagnostics Products GmbH**
**35041 Marburg (DE)**

(72) Erfinder:
• SASS, Karl
  35274 Kirchhain (DE)
• GREIS, Dirk
  35099 Burgwald (DE)
• NOAH, Michael
  35041 Marburg (DE)
• UECKERMANN, Christian
  35041 Marburg (DE)

(56) Entgegenhaltungen:
EP-A1- 1 059 522         WO-A1-03/068060
WO-A1-2006/040387     US-A1- 2010 174 491

**Beschreibung**

Die vorliegende Erfindung bezieht sich auf ein Verfahren und ein System zum Bestimmen der Konzentration von Substanzen in Körperflüssigkeiten, insbesondere von Störsubstanzen wie Bilirubin, Hämoglobin und Lipiden in Blutserum- und Blutplasmaproben.

Zahlreiche Nachweis- und Analyseverfahren zur Bestimmung physiologischer Parameter in Körperflüssigkeitsproben beruhen auf photometrischen Messprinzipien. Photometrische Verfahren ermöglichen den qualitativen und quantitativen Nachweis von Analyten in flüssigen Proben.

Die Bestimmung klinisch relevanter Parameter, wie zum Beispiel der Konzentration oder der Aktivität eines Analyten erfolgt vielfach, indem ein Aliquot einer Körperflüssigkeit eines Patienten mit einem oder mehreren Testreagenzien in vitro vermischt wird, wodurch eine biochemische Reaktion in Gang gesetzt wird, die eine messbare Veränderung einer optischen Eigenschaft des Testansatzes bewirkt. Die Photometrie untersucht und nutzt die Schwächung eines Lichtstroms beim Durchtritt durch ein absorbierendes und/oder streuendes Medium. Je nach Art der ausgelösten biochemischen oder biophysikalischen Reaktion kommen unterschiedliche photometrische Messverfahren zum Einsatz, die die Messung eines trüben flüssigen Testansatzes ermöglichen.

Hierzu können turbidimetrische Verfahren eingesetzt werden, bei denen die Trübung beziehungsweise die optische Dichte einer Lösung oder Suspension anhand der Lichtschwächung oder Extinktion eines direkt durch die Suspension hindurch tretenden Lichtstrahls gemessen wird.

Die Intensität des Lichtstrahls nimmt beim Durchtritt durch eine Messzelle beziehungsweise Küvette, die eine flüssige Probe enthält, ab. Die Verluste können durch Interaktionen des Lichtstrahls mit der in der Messzelle befindlichen Probe, beispielsweise durch Absorptions-, Diffraktions-, Streuungs- und/oder Reflexionseffekte beeinflusst werden. Im Allgemeinen können Diffraktions-, Beugungs- und Reflexionseffekte vernachlässigt beziehungsweise durch Referenzmessungen ausgeglichen werden, so dass hauptsächlich die Absorption zur Schwächung des Lichtstrahls beiträgt.

Photometrische Konzentrationsbestimmungen beruhen daher auf einer gesetzmäßigen Abhängigkeit der Extinktion beziehungsweise Absorption von der Konzentration der gelösten Stoffe und der Schichtdicke der Messzelle bei einer bestimmten Wellenlänge des eingestrahlten Lichts. Diesen Zusammenhang beschreibt das Lambert-Beersche Gesetz:

$$E(\lambda) = -\log(I/I_0) = \varepsilon(\lambda) \cdot c \cdot d \qquad\qquad (1)$$

wobei $E(\lambda)$ die von der Wellenlänge $\lambda$ des Lichtstrahls abhängige Extinktion, $I$ die Lichtintensität nach Durchtritt durch die Probe, $I_0$ die Lichtintensität vor Durchtritt durch die Probe, $\varepsilon(\lambda)$ der wellenlängenabhängige molare Extinktionskoeffizient eines durchstrahlten Stoffes, $c$ die molare Konzentration des durchstrahlten Stoffes und $d$ die durch den Lichtstrahl durchstrahlte Schichtdicke, beispielsweise der Messzelle ist.

Anhand der Extinktion $E(\lambda)$ einer Probe lässt sich die Konzentration einer Substanz in einer Lösung ermitteln. Dazu ist es erforderlich, dass zuvor die Extinktion mindestens einer Standardlösung bekannter Konzentration bestimmt wurde. Da sich die Extinktion proportional zur Konzentration verhält, kann mittels Kalibration durch Extinktionsmessungen mehrerer Standardlösungen bekannter Konzentrationen die Konzentration einer gelösten Substanz ermittelt werden.

Die Extinktion einer Probe hängt jedoch nicht nur von der Konzentration der zu bestimmenden Substanz selbst ab, sondern auch von der Art der Probenmatrix. Die Extinktionen verschiedener Substanzen verhalten sich in einem Gemisch additiv, sofern die Substanzen nicht untereinander wechselwirken. Körperflüssigkeiten, wie beispielsweise Blutplasma oder Blutserum sind jeweils komplexe Gemische und enthalten neben dem zu bestimmenden Analyten eine Vielzahl weiterer Substanzen, die die Gesamtabsorption der Probe beeinflussen.

Körperflüssigkeitsproben können jedoch in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können.

Probleme bereiten bekanntermaßen hämolytische, ikterische und/oder lipämische Serum- oder Plasmaproben, die über abnormal hohe Hämoglobin-, Bilirubin- und/oder Lipid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden solche Proben einem photometrischen Verfahren unterworfen, das der Bestimmung eines analytischen, diagnostisch relevanten Parameters dient, besteht die Gefahr einer Fehlbestimmung die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit.

Es besteht daher ein Bedarf an Verfahren zur Ermittlung der spektrometrischen Auswirkungen störender Substanzen in Körperflüssigkeitsproben.

In EP-A1-1059522, US 4,263,512, US 2009/0009750 A1 und US 2010/0174491 A1 sind verschiedene Verfahren zur Bestimmung von Bilirubin, Hämoglobin und Lipiden in Plasma- oder Serumproben beschrieben. In der EP-A1-1059522 wird beispielsweise die nach Abzug der Extinktion durch Hämoglobin und Bilirubin verbleibende Extinktion, die insbesondere auch die durch Lipide verursachte Extinktion enthält, lokal linear approximiert.

Auch das letztgenannte Verfahren hat jedoch den Nachteil, dass gerade eine vergleichsweise hohe Lipidkonzentration die Bestimmung von Bilirubin und Hämoglobin in derselben Probe beeinflussen und somit die Messwerte verfälschen kann.

Der vorliegenden Erfindung liegt demnach die Aufgabe zugrunde, ein Verfahren zur spektrophotometrischen Bestimmung mehrerer Substanzen in einer Körperflüssigkeitsprobe bereit zu stellen, das auch in Körperflüssigkeitsproben mit hohen Lipidkonzentrationen eine zuverlässige Bestimmung anderer Substanzen, wie beispielsweise Hämoglobin und Bilirubin, ermöglicht.

Diese Aufgabe wird durch das erfindungsgemäße Verfahren gelöst.

Eine Ausführungsform der vorliegenden Erfindung besteht in einem Verfahren zum Bestimmen der Konzentrationen von Substanzen in einer Körperflüssigkeitsprobe, mit den Schritten des Durchstrahlens einer Lipide und eine zweite und gegebenenfalls eine dritte Substanz enthaltenden Körperflüssigkeitsprobe mit einem Lichtstrahl bei einer Vielzahl von Lichtwellenlängen und des Erfassens einer Vielzahl von Messwerten der Extinktion der Körperflüssigkeitsprobe bei der Vielzahl von Wellenlängen, des Berechnens einer potenzfunktionalen Näherungskurve der Form

$$E(\lambda) = p \cdot \lambda^{-q}$$

für die Extinktion der Lipide (L) auf der Basis eines ersten Messwerts durch Bestimmung des Faktors p bei vorbestimmtem Exponenten q bei einer ersten Wellenlänge, bei der die nicht durch Lipide verursachte Extinktion vernachlässigbar ist, und des Bestimmens eines ersten Näherungswerts der Konzentration der zweiten Substanz auf der Basis eines zweiten Messwerts und Werten der Näherungskurve bei einer zweiten Wellenlänge.

Gemäß einer vorteilhaften Ausführungsform umfasst das Verfahren weiterhin die Schritte des Berechnens eines Extinktionswerts bei einer dritten Wellenlänge auf der Basis des ersten Näherungswerts und Werten der Näherungskurve, des Ermittelns einer Abweichung des berechneten Extinktionswerts von einem dritten Messwert bei der dritten Wellenlänge, des Korrigierens der Näherungskurve auf der Basis der ermittelten Abweichung, und des Korrigierens des ersten Näherungswerts auf der Basis des zweiten Messwerts und Werten der korrigierten Näherungskurve.

Gemäß einer weiteren vorteilhaften Ausführungsform kann die Körperflüssigkeitsprobe weiterhin eine dritte Substanz enthalten, und es können weiterhin die Schritte des Bestimmens eines zweiten Näherungswerts der Konzentration einer dritten Substanz auf der Basis des zweiten Messwerts und Werten der Näherungskurve bei einer zweiten Wellenlänge sowie eines vierten Messwerts und Werten der Näherungskurve bei einer vierten Wellenlänge, und des Korrigierens des zweiten Näherungswerts auf der Basis des zweiten Messwerts, des vierten Messwerts und Werten der korrigierten Näherungskurve, wobei der Extinktionswert zusätzlich auf der Basis des zweiten Näherungswertes berechnet wird.

In einer bevorzugten Ausführungsform können die Schritte des Berechnens des Extinktionswerts, des Ermittelns der Abweichung und des Korrigierens der Näherungskurve, des ersten Näherungswerts und des zweiten Näherungswerts so lange iteriert werden, bis die Abweichung unterhalb eines vorbestimmten Schwellwerts liegt.

Vorteilhafterweise kann die Körperflüssigkeitsprobe Blutserum oder Blutplasma umfassen. Weiterhin ist es vorteilhaft, aber nicht notwendig, wenn die zweite Substanz Hämoglobin und die dritte Substanz Bilirubin umfasst.

Gemäß einer bevorzugten Ausführungsform liegt die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm.

Gemäß einer vorteilhaften Ausführungsform beträgt der vorbestimmte Schwellwert 0,01 E.

Vorteilhafterweise kann das Korrigieren der Näherungskurve derart erfolgen, dass der erste Messwert auf der Näherungskurve liegt.

Gemäß einer vorteilhaften Ausführungsform kann das Durchstrahlen der Körperflüssigkeitsprobe mithilfe von Laser- oder Leuchtdioden und das Erfassen der Vielzahl von Messwerten mithilfe eines photometrischen Sensors erfolgen.

Die vorliegende Erfindung schafft in einer weiteren Ausführungsform ein System, z.B. ein Analysegerät, zum Bestimmen der Konzentrationen von Substanzen in einer Körperflüssigkeitsprobe, mit einer Messeinrichtung, welche dazu ausgelegt ist, Lipide und zweite Substanzen enthaltende Körperflüssigkeitsprobe mit einem Lichtstrahl bei einer Vielzahl von Lichtwellenlängen zu durchstrahlen, und eine Vielzahl von Messwerten der Extinktion der Körperflüssigkeitsprobe bei der Vielzahl von Wellenlängen zu erfassen, und einer Berechnungseinrichtung, welche dazu ausgelegt ist, eine potenzfunktionielle Näherungskurve der Form

$$E(\lambda) = p \cdot \lambda^{-q}$$

für die Extinktion der Lipide (L) auf der Basis eines ersten Messwerts durch Bestimmung des Faktors p bei vorbestimmtem Exponenten q bei einer ersten Wellenlänge, bei der die nicht durch Lipide verursachte Extinktion vernachlässigbar ist, zu berechnen, einen ersten Näherungswert der Konzentration der zweiten Substanz auf der Basis eines zweiten Messwerts und Werten der Näherungskurve bei einer zweiten Wellenlänge zu bestimmen, und einen Extinktionswert bei einer dritten Wellenlänge auf der Basis des ersten Näherungswerts und Werten der Näherungskurve zu berechnen.

Vorteilhafterweise ist die Berechnungseinrichtung weiter dazu ausgelegt, eine Abweichung des berechneten Extinktionswerts von einem dritten Messwert bei der dritten Wellenlänge zu ermitteln, die Näherungskurve auf der Basis der ermittelten Abweichung zu korrigieren und den ersten Näherungswert auf der Basis des zweiten Messwerts und Werten der korrigierten Näherungskurve zu korrigieren.

Vorteilhafterweise kann die Messeinrichtung Laser- oder Leuchtdioden und eine photometrische Sensoreinrichtung aufweisen.

In einer bevorzugten Ausführungsform kann die Berechnungseinrichtung weiterhin dazu ausgelegt sein, einen zweiten Näherungswert der Konzentration einer dritten Substanz auf der Basis des zweiten Messwerts und Werten der Näherungskurve bei einer zweiten Wellenlänge sowie eines vierten Messwerts und Werten der Näherungskurve bei einer vierten Wellenlänge zu bestimmen, und den zweiten Näherungswert auf der Basis des zweiten Messwerts und Werten der korrigierten Näherungskurve zu korrigieren, wobei der Extinktionswert zusätzlich auf der Basis des zweiten Näherungswertes berechnet wird.

Weitere Modifikationen und Variationen ergeben sich aus den Merkmalen der abhängigen Ansprüche.

Kurze Beschreibung der Figuren

Verschiedene Ausführungsformen und Ausgestaltungen der vorliegenden Erfindung werden nun in Bezug auf die beiliegenden Zeichnungen genauer beschrieben.

Fig. 1   zeigt eine schematische Darstellung eines Diagramms mit Extinktionskurven von Lipiden gemäß einer Ausführungsform der Erfindung;

Fig. 2   zeigt eine schematische Darstellung eines Diagramms mit Extinktionskurven von Körperflüssigkeitsproben gemäß einer weiteren Ausführungsform der Erfindung;

Fig. 3   zeigt eine schematische Darstellung eines Verfahrens zum Bestimmen der Konzentrationen von Substanzen in einer Körperflüssigkeitsprobe gemäß einer weiteren Ausführungsform der Erfindung;

Fig. 4   zeigt eine schematische Darstellung eines Diagramms mit Extinktionskurven von Körperflüssigkeitsproben gemäß einer weiteren Ausführungsform der Erfindung;

Fig. 5   zeigt eine schematische Darstellung eines Diagramms mit Extinktionskurven von Körperflüssigkeitsproben gemäß einer weiteren Ausführungsform der Erfindung; und

Fig. 6   zeigt eine schematische Darstellung eines Systems zum Bestimmen der Konzentrationen von Substanzen in einer Körperflüssigkeitsprobe gemäß einer weiteren Ausführungsform der Erfindung zeigt.

Die beschriebenen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt. Gleiche Bezugszeichen bezeichnen dabei gleiche oder ähnlich wirkende Komponenten.

Körperflüssigkeitsproben im Sinne der vorliegenden Erfindung können alle Proben biologischen Ursprungs sein, welche flüssige Konsistenz aufweisen und eine Vielzahl von biologisch aktiven Substanzen in verschiedenen Konzentrationen aufweisen. Beispielsweise können Körperflüssigkeitsproben Blutserum, Blutplasma, Blut, Urin, Lymphflüssigkeit, Gallenflüssigkeit oder ähnliche Flüssigkeiten aufweisen.

Photometrische Messwerte im Sinne der vorliegenden Erfindung können Messwerte sein, welche mit photometrischen Messeinrichtungen und zugehörigen Lichtquellen, insbesondere Lasern, Laserdioden, Leuchtdioden oder dergleichen aufgenommen werden können. Messeinrichtungen umfassen beispielsweise CCD-Sensoren, CMOS-Sensoren, Photosensoren oder ähnliche Einrichtungen, welche dazu geeignet sind, die Intensität eines Lichtstrahls wellenlängenabhängig zu erfassen.

Lipide im Sinne der vorliegenden Anmeldung können alle im Wesentlichen hydrophoben organischen Verbindungen umfassen, insbesondere im menschlichen oder tierischen Organismus vorkommende Verbindungen. Lipide im Sinne der Erfindung umfassen dabei insbesondere Fette bzw. Triglyceride bzw. Triacylglycerine, welche im menschlichen Körper vorkommen können.

Extinktionskurven und Extinktionswerte im Sinne der vorliegenden Erfindung können dimensionslose Größen sein, welche ein wellenlängenabhängiges Maß für die Opazität von Körperflüssigkeitsproben gegenüber dem Durchgang von Lichtstrahlen im sichtbaren, infraroten und/oder ultravioletten Wellenlängenbereich angeben. Es kann gleichermaßen auch möglich sein, dass Extinktionswerte im Bezug auf eine Einheitsdicke einer Messzelle oder Küvette, in der Körperflüssigkeitsproben während des Durchtritts von Lichtstrahlen zur Erfassung von Intensitätsmesswerten gehalten werden, angegeben werden. In diesem Fall können die Extinktionswerte eine Dimension von [1/cm] aufweisen. In jedem Fall sind die angegebenen Extinktionswerte der nachfolgenden Ausführungsformen nur beispielhafter Natur und von der Messapparatur, der Probenbeschaffenheit und der Probenzusammensetzung abhängig. Extinktionswerte werden im Folgenden jeweils mit Absorptionswerten gleichgesetzt, obwohl es dem Fachmann klar ist, dass bei dieser Betrachtung Diffraktion, Streuung und Reflexion zwar zu den Extinktionswerten beitragen, gegenüber der Absorption jedoch im betrachteten Wellenlängenbereich im Wesentlichen vernachlässigbar sind.

In Körperflüssigkeitsproben können häufig Hämoglobin, Bilirubin und Lipide, insbesondere Triacylglycerine (Triglyceride), enthalten sein. Zur Bestimmung der Hämoglobin- und Bilirubinkonzentrationen mittels photospektrometrischer Untersuchungsmethoden ist es wichtig, den Lipidanteil zu bestimmen.

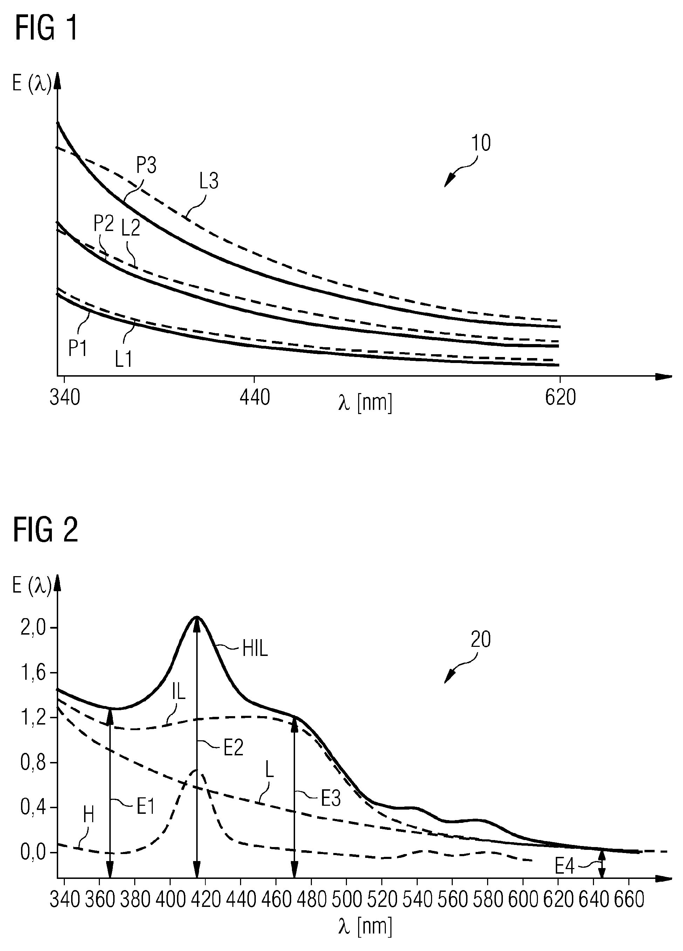

Fig. 1 zeigt eine schematische Darstellung eines Diagramms mit Extinktionskurven von Lipiden in einem Wellenlängenbereich zwischen etwa 340 nm und 620 nm. Die Extinktionskurven L1, L2 und L3 bilden jeweils künstlich angesetzte Lipid-Emulsionen (Intralipid, Lipovenös®) in den Lipid-Konzentrationen 60 mg/dl, 180 mg/dl bzw. 300 mg/dl. Zunächst lässt sich erkennen, dass die Extinktionswerte insgesamt mit der Lipidkonzentration ansteigen. Weiterhin lässt sich erkennen, dass im roten sichtbaren Spektralbereich um 600 nm bis 620 nm die Extinktion für alle Lipid-Konzentrationen geringer ist als die Extinktion im blauen sichtbaren bzw. ultravioletten Spektralbereich. Die Lipidkurven L1, L2 und L3 sind in Fig. 1 jeweils durch eine Potenzfunktion

$$E(\lambda) = p \cdot \lambda^{-q} \qquad\qquad (2)$$

angenähert worden, so dass sich die Näherungskurven P1, P2 und P3 mit jeweils angepassten Näherungsparametern p und q ergeben. Die Näherungsparameter p und q werden direkt aus den gemessenen Extinktionen berechnet und ergeben somit Erfahrungswerte für typische Größenordnungen der Parameter p und q, die im im Folgenden beschriebenen Verfahren zur Anwendung kommen können.

Für die Lipidkurve L1 der niedrigsten Konzentration stimmt die Potenzkurve P1 gut mit dem tatsächlichen Extinktionsverlauf überein. Für höhere Konzentrationen jedoch können die Näherungskurven P2 und P3 die jeweiligen Extinktionsverläufe L2 bzw. L3 nur weniger adäquat abbilden, insbesondere im blauen Wellenlängenbereich zwischen etwa 340 nm und 470 nm. Die Extinktionsverläufe L2 und L3 bilden in diesem Bereich ein gewisses Extinktionsplateau aus, was durch Mehrfachstreuung verursacht wird. Die Mehrfachstreuung kommt jedoch nur bei den in Fig. 1 dargestellten künstlichen Lipiden vor. Im Anwendungsfall kommen jedoch nur natürliche Lipide vor, bei denen es nicht zu solchen Mehrfachstreuungen kommt. Daher ermöglicht eine potenzfunktionelle Näherung trotz der in Fig. 1 dargestellten Abweichungen im tatsächlichen Anwendungsfall gute Resultate, gerade im Vergleich beispielsweise zu lokalen linearen Näherungen.

Fig. 2 zeigt eine schematische Darstellung eines Diagramms mit Extinktionskurven von Körperflüssigkeitsproben, insbesondere von Blutserum oder Blutplasma, im Wellenlängenbereich zwischen 340 nm und 660 nm. Blutserum oder Blutplasma können als emulgierte Substanzen Hämoglobin (H), Bilirubin (I) und Lipide (L) umfassen. Eine Bestimmung der Konzentration dieser Substanzen in der Körperflüssigkeitsprobe wird daher häufig auch als HIL-Check bezeichnet.

Die Extinktionskurve HIL gibt einen beispielhaften schematischen Verlauf für die wellenlängenabhängige Extinktion von Körperflüssigkeitsproben mit typischen Konzentrationen von Hämoglobin, Bilirubin und Lipiden wider. Die Extinktionskurve kann dabei in einen Hämoglobinanteil H und einen kombinierten Lipid-/Bilirubinanteil IL aufgeteilt werden, deren geschätzte Extinktionskurven als gestrichelte Kurven in Fig. 2 dargestellt sind. Der reine Lipidanteil L ist ebenfalls als gestrichelte Kurve dargestellt. Die jeweiligen Extinktionen überlagern sich additiv.

In einem roten Wellenlängenbereich zwischen etwa 610 nm und 650 nm ist die durch Hämoglobin und Bilirubin verursachte Extinktion vernachlässigbar. Die Extinktion wird somit hier weitgehend durch Lipide verursacht. Daher kann mit einer ersten Messung bei einer ersten Wellenlänge zwischen 610 nm und 650 nm, beispielsweise bei 620 nm oder 645 nm ein erster Messwert E4 ermittelt werden, mit welchem über die Abhängigkeit des Lambert-Beerschen Gesetzes die molare Lipidkonzentration $C_L$ [L/(mol*cm)] in einer ersten Näherung bestimmt werden kann:

$$c_L = E4 / \varepsilon_{L4} \; ,\qquad\qquad (3)$$

wobei $\varepsilon_{L4}$ der molare Extinktionskoeffizient von Triacylglycerinen bei der ersten Wellenlänge ist.

Vorzugsweise kann die Konzentration auch mit Hilfe eines gewichtsspezifischen Extinktionskoeffizienten bestimmt werden. Dazu wird in der Lambert-Beerschen Formel (Formel 1) die Weglänge d mit 1 mm gleichgesetzt, und aus dem Produkt des molaren Extinktionskoeffizienten $\varepsilon_{mol}$ und der Schichtdicke der Messzelle d wird der gewichtsspezifische Extinktionskoeffizient bestimmt. Dies ermöglicht eine Bestimmung der Konzentration der Substanz in [mg/dL].

In weiteren Messungen können Messwerte E2 und E3 erfasst werden, welche in Wellenlängenbereichen liegen, in denen Extinktionsmaxima von Hämoglobin beziehungsweise Bilirubin liegen. Beispielsweise kann der Messwert E2 in einem Wellenlängenbereich zwischen 410 nm und 420 nm, dem Maximum der Hämoglobinextinktion, insbesondere bei etwa 415 nm erfasst werden. Der Messwert E3 kann beispielsweise in einem Wellenlängenbereich zwischen 465 nm und 475 nm, dem Maximum der Bilirubinextinktion, insbesondere bei etwa 470 nm erfasst werden. Der Messwert E2 setzt sich aus Extinktionsanteilen zusammen, die Hämoglobin ($E_{H2}$), Bilirubin ($E_{I2}$) und Lipiden ($E_{L2}$) zugeschrieben werden können:

$$E2 = E_{H2} + E_{I2} + E_{L2} \; .\qquad\qquad (4)$$

Gleichermaßen setzt sich der Messwert E3 aus Extinktionsanteilen zusammen, die Hämoglobin ($E_{H3}$), Bilirubin ($E_{I3}$) und Lipiden ($E_{L3}$) zugeschrieben werden können:

$$E3 = E_{H3} + E_{I3} + E_{L3} \; .\qquad\qquad (5)$$

Schließlich kann ein Messwert E1 in einem Wellenlängenbereich erfasst werden, der als Kontrollbereich dienen kann, beispielsweise in einem Bereich zwischen 360 nm und 370 nm, insbesondere bei etwa 365 nm. Der Messwert E1 setzt sich aus Extinktionsanteilen zusammen, die Hämoglobin ($E_{H1}$), Bilirubin ($E_{I1}$) und Lipiden ($E_{L1}$) zugeschrieben werden können:

$$E1 = E_{H1} + E_{I1} + E_{L1} \; .\qquad\qquad (6)$$

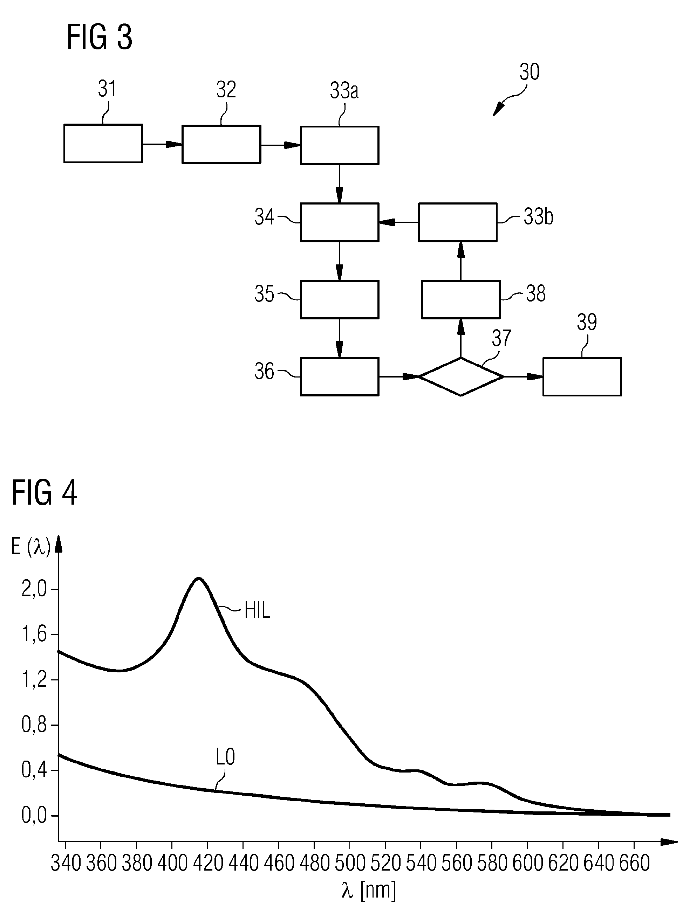

Fig. 3 zeigt nun eine schematische Darstellung eines Verfahrens 30 zum Bestimmen der Konzentrationen von Substanzen in einer Körperflüssigkeitsprobe, insbesondere von Hämoglobin, Bilirubin und Lipiden in einer Blutserum- oder Blutplasmaprobe.

In einem ersten Schritt 31 können Messwerte E1, E2, E3 und E4, wie im Zusammenhang mit Fig. 2 erläutert, erfasst werden. In einem zweiten Schritt 32 können erste Näherungsparameter $p_0$ und $q_0$ für eine erste Näherungskurve $L_0$ für die durch Lipide verursachte Extinktion mithilfe einer Regressionsanalyse bestimmt werden. Diese kann wie im Zusammenhang mit Fig. 2 erläutert, analog Gleichung (2) die Form

$$E(\lambda) = p \cdot \lambda^{-q}$$

haben. Der einzige Messwert E4 kann selbstverständlich in Schritt 32 noch nicht zu einer Bestimmung beider Variablen p und q dienen. Daher kann der Exponent $q_0$ anhand einer auf den in Fig. 1 dargestellten Referenzwerten basierenden Schätzung gebildet werden. Der Exponent $q_0$ kann demnach auf Erfahrungswerten basierend vorbestimmt werden. Da wie im Zusammenhang mit Fig. 2 beschrieben bei der ersten Wellenlänge im Bereich von 610 bis 650 nm die Extinktion durch andere Stoffe außer Lipiden vernachlässigt werden kann, kann dann bei gegebenem Exponent $q_0$ gemäß den Gleichungen (1), (2) und (3) der Koeffizient $p_0$ über den Messwert E4 bei der ersten Wellenlänge bestimmt werden. Die so ermittelte Näherungskurve mit den Parametern $p_0$ und $q_0$ kann eine erste Näherung für den Extinktionsverlauf der Extinktion von Lipiden in der Probe wiedergeben. Hierzu kann für alle Wellenlängen, in denen in Schritt 31 weitere Messwerte erfasst worden sind, in Schritt 33a der jeweilige Extinktionsanteil $E_{L1}$, $E_{L2}$, $E_{L3}$ und $E_{L4}$ (= E4), der Lipiden berechnet werden.

Wie sich in Fig. 4 erkennen lässt, ergibt sich dadurch eine erste Näherungskurve $L_0$, welche bereits eine gute Näherung an die tatsächliche Lipidextinktion bietet. Gemäß Fig. 1 kann die Näherungskurve $L_0$ insbesondere in einem blauen bzw. ultravioletten Spektralbereich flacher verlaufen als der tatsächliche Extinktionsverlauf für Lipide.

In den Schritten 34 und 35 können dann erste Näherungswerte für die Konzentrationen von Hämoglobin ($c_H$) und Bilirubin ($c_I$) auf der Basis der Messwerte E2 und E3, beispielsweise bei den Wellenlängen 415 nm und 470 nm, bestimmt werden:

$$c_I = \frac{E3 - c_H \cdot \varepsilon_{H3} - E_{L3}}{\varepsilon_{I3}} \qquad (7)$$

$$c_H = \frac{E2 - c_I \cdot \varepsilon_{I2} - E_{L2}}{\varepsilon_{H2}} \qquad (8)$$

wobei $\varepsilon_{H2}$, $\varepsilon_{H3}$, $\varepsilon_{I2}$ und $\varepsilon_{I3}$ die jeweiligen Extinktionskoeffizienten von Hämoglobin (H) und Bilirubin (I) bei den Wellenlängen der Messwerte E2 und E3 sind. Die Extinktionskoeffizienten können dabei vorab durch Referenzmessungen bestimmt werden, oder aus einem Speicher, in dem Referenzwerte abgelegt sind, für die Berechnungen abgerufen werden.

Zur Ermittlung der beiden Konzentrationen $C_I$ und $C_H$ kann das lineare Gleichungssystem der beiden Gleichungen (7) und (8) gelöst werden, so dass sich für die Konzentration von Hämoglobin (H) die Formel

$$c_H = \frac{E3 - E_{L3} - \dfrac{(E2 - E_{L2}) \cdot \varepsilon_{I3}}{\varepsilon_{I2}}}{\varepsilon_{H3} - \dfrac{\varepsilon_{H2} \cdot \varepsilon_{I3}}{\varepsilon_{I2}}} \qquad (9)$$

ergibt. Hierbei können die Extinktionswerte für die Lipide $E_{L2}$ und $E_{L3}$ gemäß Gleichung (2) mit der Näherungskurve $L_0$ bestimmt werden. Somit ergibt sich ein erster Näherungswert für die Konzentration $C_H$ von Hämoglobin. Dieser erste Näherungswert für die Konzentration $C_H$ kann dann zur Bestimmung des ersten Näherungswerts für die Konzentration $C_I$ von Bilirubin in Gleichung (7) eingesetzt werden. Hierdurch ergeben sich bereits erste, gute Näherungswerte $C_H$, $C_I$ und $C_L$ für die Konzentrationen von Hämoglobin, Bilirubin und Lipiden, die auf Basis der Potenzfunktion gemäß Gleichung (2) und dem oben beschriebenen Gleichungssystem mit den ersten Näherungswerten für die Parameter $p_0$ und $q_0$ ermittelt wurden.

Die Näherungswerte können nun aber iterativ weiter verbessert werden, wie im folgenden beschrieben wird. In einem Schritt 36 kann ein Extinktionswert $E_{HIL}$ ermittelt werden, welcher einem Näherungswert für die gesamte Extinktion bei einer Wellenlänge zwischen 360 nm und 370 nm, beispielsweise 365 nm, entspricht, d.h. in einem Bereich, in dem eine höhere Abweichung der tatsächlichen Lipidextinktion von der Näherungskurve zu erwarten ist:

$$E_{HIL} = c_H \cdot \varepsilon_{H1} + c_I \cdot \varepsilon_{I1} + E_{L1} \qquad (10)$$

Die Konzentrationen $C_H$ und $C_I$ wurden oben bestimmt, der Wert $E_L$, ergibt sich wieder aus Gleichung (2) mit den Parametern $p_0$ und $q_0$.

In einem Schritt 37 kann dann ein Vergleich zwischen dem Wert $E_{HIL}$ und dem tatsächlichen Messwert E1 bei dieser Wellenlänge durchgeführt werden, um eine Abweichung

$$\Delta E = E1 - E_{HIL} \tag{11}$$

zu erhalten. Wenn die Abweichung $\Delta E$ größer als ein vorbestimmter Schwellwert, beispielsweise 10 mE ist, kann bestimmt werden, dass die ermittelte Näherungskurve $L_0$ für die Konzentrationen der Lipide nicht hinreichend genau genug ermittelt worden ist. In diesem Fall kann in einem Schritt 38 ein Korrigieren der Näherungskurve $L_0$ erfolgen. Hierzu kann der berechnete Extinktionswert $E_{L1}$, welcher den Extinktionsanteil von Lipiden bei der Wellenlänge 365 nm beschreibt, um einen prozentualen Anteil der Abweichung $\Delta E$ korrigiert werden. Beispielsweise kann zu dem Extinktionswert $E_{L1}$ die Hälfte des Wertes der Abweichung $\Delta E$ addiert werden. Auf der Basis des korrigierten Extinktionswertes $E_{L1}$ kann dann eine korrigierte Näherungskurve $L_k$ mit den Parametern $p_k$ und $q_k$ bestimmt werden:

$$q_k = \frac{\ln E4 - \ln(E_{L1} + \Delta E/2)}{\ln \lambda(E4) - \ln \lambda(E1)} \tag{12}$$

$$p_k = \frac{E4}{\lambda(E4)^{-q_k}} \tag{13}$$

Die Gleichungen (12) und (13) ergeben sich dabei durch Einsetzen der Werte von E4 und dem korrigierten Wert E1+$\Delta E$/2 in Gleichung (2). Dies bedeutet, dass die Näherungskurve $L_0$ derart korrigiert werden kann, dass der Messwert E4, beispielsweise bei der Wellenlänge von 645 nm, weiterhin auf der korrigierten Näherungskurve $L_k$ liegt, das heißt, der Messwert E4 wird als Ankerpunkt für die Näherungskurve verwendet.

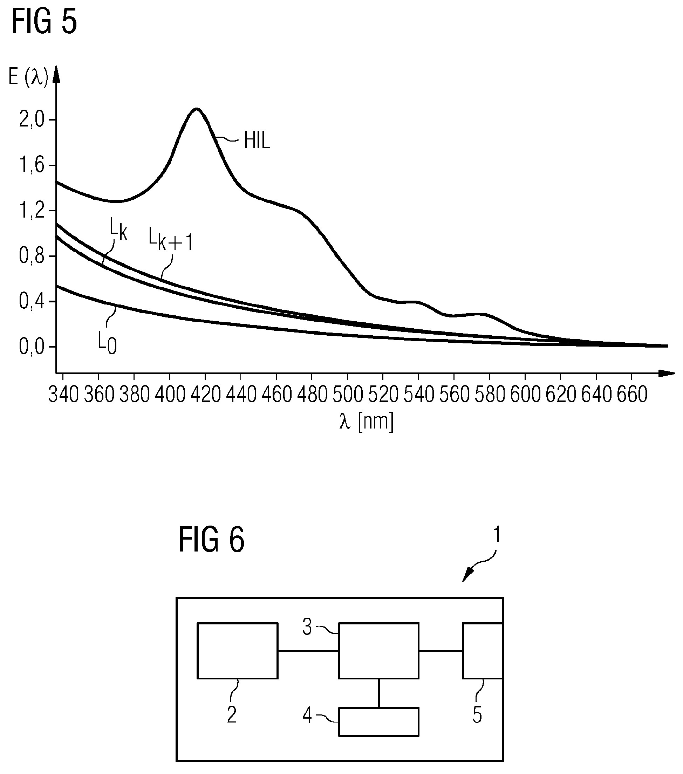

Fig. 5 zeigt eine schematische Darstellung des Diagramms aus Fig. 4, in welchem zusätzlich zu der ersten Näherungskurve $L_0$ eine korrigierte Näherungskurve $L_k$ dargestellt ist. Die korrigierte Näherungskurve $L_k$ hat insbesondere im blauen bzw. ultravioletten Wellenlängenbereich einen steileren Verlauf als die erste Näherungskurve $L_0$ und ist somit besser geeignet, den tatsächlichen Extinktionsanteil der in der Probe vorhandenen Lipide abzubilden.

In Schritt 33b können dann analog zu den Berechnungen in Schritt 33a die jeweiligen Extinktionsanteile $E_{L1}$, $E_{L2}$, $E_{L3}$ und $E_{L4}$ (= E4), der Lipide auf der Basis der korrigierten Näherungskurve $L_k$ errechnet werden. Das Verfahren kann so lange mit den Schritten 34, 35, 36, 37, 38 und 33b iteriert werden, bis in Schritt 37 bestimmt wird, dass die Abweichung einen vorbestimmten Schwellwert unterschreitet. In diesem Fall können in Schritt 39 die korrigierten Näherungswerte für die Konzentrationen der Substanzen in der Körperflüssigkeitsprobe ausgegeben werden.

In Fig. 5 ist hierzu beispielhaft eine in einem nachfolgenden Iterationsschritt korrigierte Näherungskurve $L_{k+1}$ dargestellt, welche gegenüber der korrigierten Näherungskurve $L_k$ eine bessere Annäherung an den tatsächlichen Extinktionsanteil der in der Probe vorhandenen Lipide darstellt.

Fig. 6 zeigt eine schematische Darstellung eines Systems 1 zum Bestimmen der Konzentrationen von Substanzen in einer Körperflüssigkeitsprobe, insbesondere zum Durchführen des in Fig. 3 gezeigten Verfahrens 30. Das System 1 umfasst eine Messeinrichtung 2, eine Berechnungseinrichtung 3, einen Speicher 4 und eine Ausgabeeinrichtung 5.

Die Messeinrichtung 2 kann dazu ausgelegt sein, eine erste und zweite Substanzen enthaltende Körperflüssigkeitsprobe mit einem Lichtstrahl bei einer Vielzahl von Lichtwellenlängen zu durchstrahlen, und eine Vielzahl von Messwerten der Extinktion der Körperflüssigkeitsprobe bei der Vielzahl von Wellenlängen zu erfassen. Dazu kann die Messeinrichtung 2 beispielsweise Leucht- oder Laserdioden und entsprechende photometrische Sensoreinrichtungen aufweisen.

Die Berechnungseinrichtung 3 kann dazu ausgelegt sein, die Schritte 32, 33a, 34, 35, 36, 37, 38, 33b und 39 des Verfahrens 30 in Fig. 3 durchzuführen. Insbesondere können die ermittelten Näherungswerte für die Konzentrationen der Substanzen über die Ausgabeeinrichtung 5 an einen Nutzer des Systems 1 ausgegeben werden.

Der Speicher 4 kann dazu ausgelegt sein, vorbestimmte Werte für den Schwellwert und/oder Extinktionskoeffizienten zu speichern, die die Berechungseinrichtung 2 bei Bedarf abrufen kann.

**Patentansprüche**

1. Verfahren zum Bestimmen der Konzentrationen von Lipiden (L) und mindestens einer weiteren Substanz (H) in einer Körperflüssigkeitsprobe, mit den Schritten:

a) Durchstrahlen (31) der Körperflüssigkeitsprobe mit Licht bei einer Vielzahl von Wellenlängen und
b) Erfassen einer Vielzahl von Messwerten der Extinktion der Körperflüssigkeitsprobe bei der Vielzahl von Wellenlängen;

**gekennzeichnet durch** die Schritte:

c) Erfassen eines ersten Messwertes (E4) bei einer ersten Wellenlänge, bei der die nicht **durch** Lipide verursachte Extinktion vernachlässigbar ist, und Bestimmen der Konzentration ($c_L$) der Lipide (L) **durch** Division des Messwerts (E4) mit dem für Lipide (L) spezifischen Extinktionskoeffizienten;
d) Berechnen (32) einer potenzfunktionellen Näherungskurve ($L_0$) der Form

$$E(\lambda) = p \cdot \lambda^{-q}$$

für die Extinktion der Lipide (L) auf der Basis des ersten Messwerts (E4) **durch** Bestimmung des Faktors p bei vorbestimmtem Exponenten q;
e) Bestimmen (34) eines ersten Näherungswerts ($c_H$) der Konzentration der zweiten Substanz (H) auf der Basis eines zweiten Messwerts (E2) und Werten der Näherungskurve ($L_0$) bei einer zweiten Wellenlänge;

2. Verfahren nach Anspruch 1, mit den Schritten:

f) Berechnen eines Extinktionswerts ($E_{HIL}$) bei einer dritten Wellenlänge auf der Basis des ersten Näherungswerts ($c_H$) und Werten der Näherungskurve ($L_0$);
g) Ermitteln (36) einer Abweichung ($\Delta E$) des berechneten Extinktionswerts ($E_{HIL}$) von einem dritten Messwert (E1) bei der dritten Wellenlänge;
h) Korrigieren (38) der Näherungskurve ($L_k$) auf der Basis der ermittelten Abweichung ($\Delta E$); und
i) Bestimmen der Konzentration ($c_H$) der zweiten Substanz (H) durch Korrigieren des ersten Näherungswerts ($c_H$) auf der Basis des zweiten Messwerts (E2) und Werten der korrigierten Näherungskurve ($L_k$).

3. Verfahren nach Anspruch 2, wobei ferner eine dritte Substanz (I) in der Körperflüssigkeitsprobe bestimmt wird, ferner mit den Schritten:

j) Bestimmen (35) eines zweiten Näherungswerts ($c_I$) der Konzentration der dritten Substanz (I) auf der Basis des zweiten Messwerts (E2) und Werten der Näherungskurve ($L_0$) bei der zweiten Wellenlänge sowie eines vierten Messwerts (E3) und Werten der Näherungskurve ($L_0$) bei einer vierten Wellenlänge; und
k) Bestimmen der Konzentration ($c_I$) der dritten Substanz (I) durch Korrigieren des zweiten Näherungswerts ($C_I$) auf der Basis des zweiten Messwerts (E2), des vierten Messwerts (E3) und Werten der korrigierten Näherungskurve ($L_k$),

wobei der Extinktionswert ($E_{HIL}$) zusätzlich auf der Basis des zweiten Näherungswertes ($c_I$) berechnet wird.

4. Verfahren nach Anspruch 3, wobei die Schritte des Berechnens des Extinktionswerts, des Ermittelns der Abweichung und des Korrigierens der Näherungskurve, des ersten Näherungswerts und des zweiten Näherungswerts so lange iteriert werden, bis die Abweichung ($\Delta E$) unterhalb eines vorbestimmten Schwellwerts liegt.

5. Verfahren nach Anspruch 4, wobei die zweite Substanz (H) Hämoglobin und die dritte Substanz (I) Bilirubin umfasst.

6. Verfahren nach einem der Ansprüche 3 bis 5, wobei die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm liegt.

7. Verfahren nach Anspruch 4, wobei der vorbestimmte Schwellwert 10 mE beträgt.

8. Verfahren nach einem der Ansprüche 2 bis 7, wobei das Korrigieren der Näherungskurve derart erfolgt, dass der erste Messwert (E4) auf der Näherungskurve ($L_0$; $L_k$) liegt.

9. Verfahren nach einem der Ansprüche 1 bis 8, wobei das Durchstrahlen der Körperflüssigkeitsprobe mithilfe von Laser- oder Leuchtdioden und das Erfassen der Vielzahl von Messwerten (E1; E2; E3; E4) mithilfe eines photometrischen Sensors erfolgt.

10. System (1) zum Bestimmen der Konzentrationen von Substanzen in einer Körperflüssigkeitsprobe, mit:

i. einer Messeinrichtung (2), welche dazu ausgelegt ist, eine Körperflüssigkeitsprobe mit Lichtstrahlen mit einer Vielzahl von Wellenlängen zu durchstrahlen, und eine Vielzahl von Messwerten der Extinktion der Körperflüssigkeitsprobe bei der Vielzahl von Wellenlängen zu erfassen; und

ii. einer Berechnungseinrichtung (3), welche dazu ausgelegt ist, eine potenzfunktionelle Näherungskurve ($L_0$) der Form

$$E(\lambda) = p \cdot \lambda^{-q}$$

für die Extinktion der Lipide (L) auf der Basis eines ersten Messwerts durch Bestimmung des Faktors p bei vorbestimmtem Exponenten q bei einer ersten Wellenlänge, bei der die nicht durch Lipide verursachte Extinktion vernachlässigbar ist, zu berechnen, und einen ersten Näherungswert ($c_H$)der Konzentration einer zweiten Substanz (H) auf der Basis eines zweiten Messwerts und Werten der Näherungskurve bei einer zweiten Wellenlänge zu bestimmen.

11. System (1) nach Anspruch 10, wobei die Berechnungseinrichtung (3) dazu ausgelegt ist, einen Extinktionswert ($E_{HIL}$) bei einer dritten Wellenlänge auf der Basis des ersten Näherungswerts ($c_H$) und Werten der Näherungskurve ($L_0$) zu berechnen, eine Abweichung ($\Delta E$) des berechneten Extinktionswerts ($E_{HIL}$) von einem dritten Messwert (E1) bei der dritten Wellenlänge zu ermitteln, die Näherungskurve ($L_k$) auf der Basis der ermittelten Abweichung ($\Delta E$) zu korrigieren und den ersten Näherungswert ($c_H$) auf der Basis des zweiten Messwerts (E2) und Werten der korrigierten Näherungskurve ($L_k$) zu korrigieren.

12. System (1) nach Anspruch 11, wobei die Messeinrichtung (2) Laser- oder Leuchtdioden und eine photometrische Sensoreinrichtung aufweist.

13. System (1) nach einem der Ansprüche 11 und 12, wobei die Berechnungseinrichtung (3) weiterhin dazu ausgelegt ist, einen zweiten Näherungswert der Konzentration einer dritten Substanz (I) auf der Basis des zweiten Messwerts und Werten der Näherungskurve bei einer zweiten Wellenlänge sowie eines vierten Messwerts und Werten der Näherungskurve bei einer vierten Wellenlänge zu bestimmen, und den zweiten Näherungswert auf der Basis des zweiten Messwerts und Werten der korrigierten Näherungskurve zu korrigieren, wobei der Extinktionswert zusätzlich auf der Basis des zweiten Näherungswertes berechnet wird.

14. System (1) nach Anspruch 13, wobei die Körperflüssigkeitsprobe Blutserum oder Blutplasma, die zweite Substanz (H) Hämoglobin und die dritte Substanz (I) Bilirubin umfasst.

15. System (1) nach einem der Ansprüche 13 und 14, wobei die erste Wellenlänge im Bereich zwischen 610 nm und 650 nm, die zweite Wellenlänge im Bereich zwischen 410 nm und 420 nm, die dritte Wellenlänge im Bereich zwischen 360 nm und 370 nm und die vierte Wellenlänge im Bereich zwischen 465 nm und 475 nm liegt.

**Claims**

1. Method for determining the concentrations of lipids (L) and at least one further substance (H) in a body fluid sample, comprising the steps of:

a) irradiating (31) light through the body fluid sample at a multiplicity of wavelengths and
b) capturing a multiplicity of measured values of the absorbance of the body fluid sample at the multiplicity of wavelengths;

**characterized by** the steps of:

c) capturing a first measured value (E4) at a first wavelength at which the absorbance not caused by lipids is negligible, and determining the concentration ($c_L$) of the lipids (L) by dividing the measured value (E4) by the extinction coefficient specific for lipids (L);

d) calculating (32) a power-function approximation curve ($L_0$) of the form

$$E(\lambda) \;=\; p \cdot \lambda^{-q}$$

for the absorbance of the lipids (L) on the basis of the first measured value (E4) by determining the factor p at predetermined exponent q;

e) determining (34) a first approximate value ($C_H$) of the concentration of the second substance (H) on the basis of a second measured value (E2) and values of the approximation curve ($L_0$) at a second wavelength.

2. Method according to Claim 1, comprising the steps of:

f) calculating an absorbance value ($E_{HIL}$) at a third wavelength on the basis of the first approximate value ($C_H$) and values of the approximation curve ($L_0$);

g) ascertaining (36) a deviation ($\Delta E$) of the calculated absorbance value ($E_{HIL}$) from a third measured value (EI) at the third wavelength;

h) correcting (38) the approximation curve ($L_k$) on the basis of the ascertained deviation ($\Delta E$); and

i) determining the concentration ($C_H$) of the second substance (H) by correcting the first approximate value ($C_H$) on the basis of the second measured value (E2) and values of the corrected approximation curve ($L_k$).

3. Method according to Claim 2, wherein a third substance (I) in the body fluid sample is additionally determined, additionally comprising the steps of:

j) determining (35) a second approximate value ($C_I$) of the concentration of the third substance (I) on the basis of the second measured value (E2) and values of the approximation curve ($L_0$) at the second wavelength and also of a fourth measured value (E3) and values of the approximation curve ($L_0$) at a fourth wavelength; and

k) determining the concentration ($c_I$) of the third substance (I) by correcting the second approximate value ($c_I$) on the basis of the second measured value (E2), the fourth measured value (E3) and values of the corrected approximation curve ($L_k$),

wherein the absorbance value ($E_{HIL}$) is additionally calculated on the basis of the second approximate value ($C_I$).

4. Method according to Claim 3, wherein the steps of calculating the absorbance value, of ascertaining the deviation, and of correcting the approximation curve, the first approximate value and the second approximate value are iterated until the deviation ($\Delta E$) is below a predetermined threshold.

5. Method according to Claim 4, wherein the second substance (H) comprises hemoglobin and the third substance (I) comprises bilirubin.

6. Method according to any of Claims 3 to 5, wherein the first wavelength is within the range between 610 nm and 650 nm, the second wavelength is within the range between 410 nm and 420 nm, the third wavelength is within the range between 360 nm and 370 nm, and the fourth wavelength is within the range between 465 nm and 475 nm.

7. Method according to Claim 4, wherein the predetermined threshold is 10 mE.

8. Method according to any of Claims 2 to 7, wherein the approximation curve is corrected in such a way that the first measured value (E4) lies on the approximation curve ($L_0$; $L_k$).

9. Method according to any of Claims 1 to 8, wherein irradiation through the body fluid sample is achieved using laser or light-emitting diodes and the multiplicity of measured values (EI; E2; E3; E4) is captured using a photometric sensor.

10. System (1) for determining the concentrations of substances in a body fluid sample, comprising:

i. a measurement device (2) designed for irradiating light beams having a multiplicity of wavelengths through a body fluid sample, and for capturing a multiplicity of measured values of the absorbance of the body fluid sample at the multiplicity of wavelengths; and
ii. a calculation device (3) designed for calculating a power-function approximation curve (L₀) of the form

$$E(\lambda) = p \cdot \lambda^{-q}$$

for the absorbance of the lipids (L) on the basis of a first measured value by determining the factor p at predetermined exponent q at a first wavelength at which the absorbance not caused by lipids is negligible, and for determining a first approximate value ($C_H$) of the concentration of a second substance (H) on the basis of a second measured value and values of the approximation curve at a second wavelength.

11. System (1) according to Claim 10, wherein the calculation device (3) is designed for calculating an absorbance value ($E_{HIL}$) at a third wavelength on the basis of the first approximate value ($C_H$) and values of the approximation curve (L₀), for ascertaining a deviation (ΔE) of the calculated absorbance value ($E_{HIL}$) from a third measured value (El) at the third wavelength, for correcting the approximation curve ($L_k$) on the basis of the ascertained deviation (ΔE), and for correcting the first approximate value ($C_H$) on the basis of the second measured value (E2) and values of the corrected approximation curve ($L_k$).

12. System (1) according to Claim 11, wherein the measurement device (2) comprises laser or light-emitting diodes and a photometric sensor device.

13. System (1) according to either of Claims 11 and 12, wherein the calculation device (3) is additionally designed for determining a second approximate value of the concentration of a third substance (I) on the basis of the second measured value and values of the approximation curve at a second wavelength and also of a fourth measured value and values of the approximation curve at a fourth wavelength, and for correcting the second approximate value on the basis of the second measured value and values of the corrected approximation curve, wherein the absorbance value is additionally calculated on the basis of the second approximate value.

14. System (1) according to Claim 13, wherein the body fluid sample comprises blood serum or blood plasma, the second substance (H) comprises hemoglobin and the third substance (I) comprises bilirubin.

15. System (1) according to either of Claims 13 and 14, wherein the first wavelength is within the range between 610 nm and 650 nm, the second wavelength is within the range between 410 nm and 420 nm, the third wavelength is within the range between 360 nm and 370 nm, and the fourth wavelength is within the range between 465 nm and 475 nm.

**Revendications**

1. Procédé de détermination des concentrations de lipides (L ) et d'au moins une autre substance ( H ) dans un échantillon de liquide corporel, comprenant les stades :

a) on fait traverser ( 31 ) l'échantillon de liquide corporel par de la lumière à une pluralité de longueurs d'onde et
b) on relève une pluralité de valeurs de mesure de l'extinction de l'échantillon de liquide corporel à la pluralité de longueurs d'onde ;

**caractérisé par** les stades :

c) on relève une première valeur ( E4 ) de mesure à une première longueur d'onde, à laquelle l'extinction qui n'est pas provoquée par des liquides est négligeable, et on détermine la concentration ( $C_L$ ) des lipides ( L ) en divisant la valeur ( E4 ) de mesure par le coefficient d'extinction spécifique pour des lipides ( L ) ;
d) on calcule ( 32 ) une courbe ( L₀ ) d'approximation à fonction puissance de la forme

fonction puissance de la forme

$$E(\lambda) = p \cdot \lambda^{-q}$$

pour l'extinction des lipides ( L ) sur la base de la première valeur ( E4 ) de mesure en déterminant le facteur p pour des exposants q déterminés à l'avance ;
e) on détermine ( 34 ) une première valeur ( $C_H$ ) d'approximation de la concentration de la deuxième substance ( H ) sur la base d'une deuxième valeur ( E2 ) de mesure et de valeurs de la courbe ( $L_0$ ) d'approximation à une deuxième longueur d'onde.

2.  Procédé suivant la revendication 1, comprenant les stades :

    f) on calcule une valeur ( $E_{HIL}$ ) d'extinction à une troisième longueur d'onde sur la base de la première valeur ( $C_H$ ) d'approximation et de valeurs de la courbe ( $L_0$ ) d'approximation ;
    g) on détermine ( 36 ) un écart ( $\Delta E$ ) de la valeur ( $E_{HIL}$ ) d'extinction calculée à une troisième valeur ( E1 ) à la troisième longueur d'onde ;
    h) on corrige ( 38 ) la courbe ( $L_K$ ) d'approximation sur la base de l'écart ( $\Delta E$ ) déterminé et
    i) on détermine la concentration ( $C_H$ ) de la deuxième substance ( H ) en corrigeant la première valeur ( $C_H$ ) d'approximation sur la base de la deuxième valeur ( E2 ) de mesure et de valeurs de la courbe ( $L_K$ ) d'approximation corrigée.

3.  Procédé suivant la revendication 2, dans lequel on détermine, en outre, une troisième substance ( I ) dans l'échantillon de liquide corporel, comprenant, en outre, par les stades :

    j) on détermine ( 35 ) une deuxième valeur ( $C_I$ ) d'approximation de la concentration de la troisième substance ( I ) sur la base de la deuxième valeur ( E2 ) de mesure et de valeurs de la courbe ( $L_0$ ) d'approximation à la deuxième longueur d'onde ainsi que d'une quatrième valeur ( E3 ) de mesure et de valeurs de la courbe ( $L_0$ ) d'approximation à une quatrième longueur d'onde et
    k) on détermine la concentration ( $C_I$ ) de la troisième substance ( I ) en corrigeant la deuxième valeur ( $C_I$ ) d'approximation sur la base de la deuxième valeur ( E2 ) de mesure, de la quatrième valeur ( E3 ) de mesure et de valeurs de la courbe ( $L_K$ ) d'approximation corrigée,

    dans lequel on calcule la valeur ( $E_{HIL}$ ) d'extinction supplémentairement sur la base de la deuxième valeur ( $C_I$ ).

4.  Procédé suivant la revendication 3, dans lequel on répète par itération les stades du calcul de la valeur d'extinction, de la détermination de l'écart et de la correction de la courbe d'approximation, de la première valeur d'approximation et de la deuxième valeur d'approximation jusqu'à ce que l'écart ( $\Delta E$ ) devienne inférieur à une valeur de seuil déterminée à l'avance.

5.  Procédé suivant la revendication 4, dans lequel la deuxième substance ( H ) comprend de l'hémoglobine et la troisième substance ( I ) de la bilirubine.

6.  Procédé suivant l'une des revendications 3 à 5, dans lequel la première longueur est dans la plage comprise entre 610 nm et 650 nm, la deuxième longueur d'onde dans la plage comprise entre 410 nm et 420 nm, la troisième longueur d'onde dans la plage comprise entre 360 nm et 370 nm et la quatrième longueur d'onde dans la plage comprise entre 465 nm et 475 nm.

7.  Procédé suivant la revendication 4, dans lequel la valeur de seuil déterminée à l'avance est de 10 mE.

8.  Procédé suivant l'une des revendications 2 à 7, dans lequel on effectue la correction de la courbe d'approximation de manière à ce que la première valeur ( E4 ) de mesure soit sur la courbe ( $L_0$, $L_K$ ) d'approximation.

9.  Procédé suivant l'une des revendications 1 à 8, dans lequel on fait traverser l'échantillon de liquide corporel par de la lumière à l'aide de diodes laser ou de diodes électroluminescentes et on effectue le relevé de la pluralité de valeurs ( E1, E2, E3, E4 ) de mesure à l'aide d'une sonde photométrique.

**10.** Système ( 1 ) de détermination des concentrations de substances dans un échantillon de liquide corporel comprenant :

i. un dispositif ( 2 ) de mesure, qui est conçu pour faire passer à travers un échantillon de liquide corporel des faisceaux lumineux d'une pluralité de longueurs d'onde et pour relever une pluralité de valeurs de mesure de l'extinction de l'échantillon de liquide corporel à la pluralité de longueurs d'onde et

ii. un dispositif ( 3 ) de calcul, qui est conçu pour calculer une courbe ($L_0$) d'approximation à fonction puissance de la forme

$$E(\lambda) = p \cdot \lambda^{-q}$$

pour l'extinction des lipides ( L ) sur la base d'une première valeur de mesure en déterminant le facteur p à des exposants q déterminés à l'avance, à une première longueur d'onde, à laquelle l'extinction qui n'est pas provoquée par des lipides est négligeable et pour déterminer une première valeur ($c_H$) d'approximation de la concentration d'une deuxième substance ( H ) sur la base d'une deuxième valeur de mesure et de valeurs de la courbe d'approximation, à une deuxième longueur d'onde.

**11.** Système ( 1 ) suivant la revendication 10, dans lequel le dispositif ( 3 ) de calcul est conçu pour calculer une valeur ($E_{HIL}$) d'extinction à une troisième longueur d'onde sur la base de la première valeur ($c_H$) d'approximation et de valeurs de la courbe ($L_0$) d'approximation pour déterminer un écart ($\Delta E$) de la valeur ($E_{HIL}$) d'extinction calculée à une troisième valeur ( E1 ) de mesure à la troisième longueur d'onde, pour corriger la courbe ($L_K$) d'approximation sur la base de l'écart ($\Delta E$) déterminé et pour corriger la première valeur ($c_H$) d'approximation sur la base de la deuxième valeur ( E2 ) de mesure et de valeur, de la courbe ($L_K$) d'approximation corrigée.

**12.** Système ( 1 ) suivant la revendication 11, dans lequel le dispositif ( 2 ) de mesure a des diodes laser ou des diodes électroluminescentes et un dispositif à sonde photométrique.

**13.** Système ( 1 ) suivant l'une des revendications 11 et 12, dans lequel le dispositif ( 3 ) de calcul est conçu, en outre, pour déterminer une deuxième valeur d'approximation de la concentration d'une troisième substance ( I ) sur la base de la deuxième valeur de mesure et de valeurs de la courbe d'approximation à une deuxième longueur d'onde, ainsi que d'une quatrième valeur de mesure et de valeurs de la courbe d'approximation à une quatrième longueur d'onde et pour corriger la deuxième valeur d'approximation sur la base de la deuxième valeur de mesure et de valeurs de la courbe d'approximation corrigée, la valeur d'extinction étant calculée supplémentairement sur la base de la deuxième valeur d'approximation.

**14.** Système ( 1 ) suivant la revendication 13, dans lequel l'échantillon de liquide corporel comprend du sérum sanguin ou du plasma sanguin, la deuxième substance ( H ) de l'hémoglobine et la troisième substance ( I ) de la bilirubine.

**15.** Système ( 1 ) suivant l'une des revendications 13 et 14, dans lequel la première longueur d'onde est dans la plage comprise entre 610 nm et 650 nm, la deuxième longueur d'onde dans la plage comprise entre 410 nm et 420 nm, la troisième longueur d'onde dans la plage comprise entre 360 nm et 370 nm et la quatrième longueur d'onde dans la plage comprise entre 465 nm et 475 nm.

## FIG 1

## FIG 2

## FIG 3

## FIG 4

## FIG 5

## FIG 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1059522 A1
- US 4263512 A
- US 20090009750 A1
- US 20100174491 A1